# FASCICULE DE BREVET EUROPEEN

(11) **EP 1 246 657 B1**
(45) Date de publication et mention de la délivrance du brevet: **29.09.2004**
(21) Numéro de dépôt: 00988942.9
(22) Date de dépôt: 18.12.2000
(51) Int. Cl.: A61M 5/30

(54) **SERINGUE SANS AIGUILLE FONCTIONNANT PAR EFFET TUBE A CHOC, AVEC MAINTIEN PREALABLE DU PRINCIPE ACTIF SUR LE COTE**
NADELLOSE SPRITZE MIT IN STRÖMUNG BEI STOSSWELLENROHREFFEKT GETRAGENEN WIRKSTOFFPARTIKELN MIT SEITLICHEM VORLAGER
NEEDLELESS SYRINGE OPERATING BY IMPACT TUBE EFFECT, WITH PRIOR LATERAL RETENTION OF ACTIVE PRINCIPLE

(30) Priorité: 27.12.1999 FR 9916536
(43) Date de publication de la demande: 09.10.2002
(73) Titulaire: Crossject, 75181 Paris Cédex 04 (FR)
(72) Inventeur: ALEXANDRE, Patrick, F-70100 Gray (FR); BAUD, Georges, F-83260 La Crau (FR); DELANNOY, Guy, F-33160 Saint Medard en Jalles (FR); ROLLER, Denis, F-91590 La Ferte Alais (FR)
(74) Mandataire: Waligorski, Carol
(86) Numéro de dépôt international: PCT/FR2000/003567
(87) Numéro de publication internationale: WO 2001/047585

(56) Documents cités:
- WO-A-94/24263
- WO-A-99/01169
- US-A- 3 763 859
- US-A- 3 788 315
- US-A- 4 089 334
- US-A- 5 865 796

## Description

Le domaine technique de l'invention est celui des seringues sans aiguille utilisées pour les injections intradermiques, sous-cutanées ou intramusculaires de divers principes actifs pulvérulents, à usage thérapeutique pour la médecine humaine ou vétérinaire.

Plus spécifiquement, l'invention se rapporte à une seringue sans aiguille mettant en oeuvre un générateur de gaz destiné à créer une onde de pression pour éjecter les particules de principe actif. Un opercule claquable, placé sur le chemin des gaz, permet d'obtenir le niveau de pression seuil permettant d'éjecter les particules avec une vitesse suffisamment élevée. En fait, la libération soudaine des gaz crée dans la seringue une onde de choc et c'est cette onde qui va porter et accélérer les particules afin de les expulser. La spécificité de l'invention réside dans le fait que les particules initialement isolées du circuit principal d'éjection de la seringue, sont d'abord. amenées sur le chemin des gaz juste avant d'être soufflées par l'onde de choc sur la peau du patient.

Les seringues sans aiguille fonctionnant par génération d'un choc pour emporter les particules solides de principe actif existent déjà et ont fait l'objet de plusieurs brevets. On peut citer en particulier le brevet WO 94/24263 qui décrit une seringue sans aiguille fonctionnant par libération d'une réserve de gaz comprimé pour entraîner les particules solides de principe actif. Dans ce brevet, l'une des caractéristiques principales est que les particules sont maintenues en permanence sur le chemin des gaz, entre deux membranes soufflables. Il n'est jamais envisagé de stocker les particules en dehors du circuit principal d'éjection des particules. Il faut également mentionner le brevet WO 99/01169 qui se réfère à une seringue sans aiguille fonctionnant avec une capsule destinée à contenir le principe actif, et constituée de deux éléments couplés dont l'un est mobile. Sous l'effet de l'arrivée des gaz sous pression, l'élément mobile de la capsule se déplace créant ainsi un passage à travers ladite capsule. Le principe actif se retrouve alors entraîné dans ce passage par les gaz sous pression et est ensuite soufflé vers la peau du patient. Dans ce brevet, le principe actif est toujours maintenu dans le chemin des gaz et la création du passage pour permettre la fuite du principe actif s'effectue par le déplacement, le long de laxe de la seringue, de l'un des éléments constitutifs du système de retenue des particules.

Il existe par ailleurs des dispositifs, comme celui décrit par exemple dans le brevet US 5 478 744, qui permettent de bombarder des cultures cellulaires avec des particules inertes ou biologiquement actives, et dont le principe de fonctionnement repose sur la libération d'un gaz sous pression dans un tube qui peut être alimenté latéralement par des particules. Mais il faut constater que ce sont des dispositifs de laboratoire, certes performants, mais lourds et encombrants et dont les caractéristiques ne sont pas directement transposables à un objet léger, de petite taille comme une seringue sans aiguille.

La seringue sans aiguille selon l'invention est apte à éjecter des particules solides de principe actif par l'effet d'un choc produit par un gaz sous pression, en permettant les deux étapes suivantes : d'abord, les particules qui sont situées dans des logements extérieurs au tube d'éjection sont libérées dans ledit tube, puis l'onde de choc passe dans le tube emportant avec elle les particules déjà en mouvement. Ainsi, la seringue sans aiguille selon l'invention dispose d'un système d'entraînement des particules de faible, encombrement, efficace et performant, tout en évitant de maintenir en permanence lesdites particules sur le chemin des gaz. De cette manière, les particules de principe actif se retrouvent isolées et complètement inaccessibles, renforçant ainsi la fiabilité de la seringue. De plus, les particules étant soufflées librement dans le tube d'éjection avant le passage de l'onde de choc, le risque d'éjecter des fragments parasites provenant, par exemple, de membranes de retenue des particules de principe actif est nul.

L'objet de la présente invention concerne une seringue sans aiguille comme définie dans la revendication 1. Préférentiellement, la seringue sans aiguille selon l'invention est particulièrement adaptée à l'injection de principe actif pulvérulent ou sous forme de poudre sèche. La principale caractéristique de la seringue sans aiguille selon l'invention est de permettre l'arrivée des particules de principe actif dans le tube d'éjection, juste avant que le choc engendré par les gaz dans ledit tube ne les emporte. Une telle seringue trouve sa pleine efficacité lorsque l'intervalle de temps, entre le moment où les particules sont libérées dans le tube d'éjection et le moment où l'onde de choc interagit avec elles, est faible, c'est à dire de l'ordre de la milliseconde ou de quelques millisecondes. En effet, au moment où elles sont soufflées, les particules doivent être encore en phase de dispersion dans la seringue, sous la forme d'un « nuage » plus ou moins homogène, sans qu'elles n'aient encore eu le temps de se recomposer par simple gravité. Cette condition est nécessaire pour assurer une injection uniforme sans privilégier involontairement de zones particulières d'impact sur la peau. De façon préférentielle, le générateur de gaz est un générateur pyrotechnique comprenant une charge pyrotechnique et un dispositif d'initiation. Selon un mode de réalisation préféré de l'invention, le dispositif d'initiation comprend un dispositif de percussion et une amorce couramment utilisés en industrie pyrotechnique. Mais il est également possible d'initier la charge pyrotechnique par d'autres moyens, et notamment, ceux impliquant soit un cristal piézo-électrique, soit un rugueux ou même une pile. La seringue sans aiguille selon l'invention peut également fonctionner avec un générateur de gaz constitué par une réserve de gaz sous pression. Avantageusement, le déclencheur est situé à l'une des extrémités de la seringue sous la forme d'un bouton poussoir pour faciliter sa préhension et son actionnement.

Selon un premier mode de réalisation préféré de l'invention, les particules sont situées dans au moins un logement inamovible extérieur au conduit du tube, chaque logement étant obturé par le piston positionné dans ledit tube, ledit piston pouvant se déplacer sous l'effet des gaz, pour ouvrir chaque logement et libérer les particules dans le tube. De façon avantageuse, un dispositif de propulsion permet aux particules libérées par le piston d'être poussées dans le tube à partir de leur logement. En effet, l'objectif étant d'injecter la plus grande quantité de principe actif présente dans la seringue, il est indispensable d'assurer la vidange complète de tous les logements afin que toutes les particules soient en position d'être éjectées. Selon un mode de réalisation préféré de l'invention, le dispositif de propulsion est réalisé par prélèvement de gaz grâce à au moins une tubulure reliant la zone de la chambre située à proximité du générateur de gaz aux logements des particules et, préférentiellement, il existe autant de tubulures que de logements de particules. De cette manière, le dispositif de propulsion des particules s'appuie sur une source énergétique préexistante ne nécessitant pas un encombrement accru dû à un dispositif supplémentaire, isolé et autonome. De façon avantageuse, les particules sont logées dans la partie terminale de chaque tubulure, contre le piston. Plus précisément, les particules sont logées dans un espace délimité, d'une part, par le piston et, d'autre part, par un film mince transversal situé dans la tubulure. Ce film mince peut être constitué, par exemple, par une membrane soufflable de très faible épaisseur, ne pouvant pas résister à une pression supérieure à 10 bars, ou bien par une membrane poreuse.

Selon un autre mode de réalisation de l'invention, les particules sont bloquées dans leur logement, entre le piston et un ressort précontraint, de sorte que lorsque le piston ne bouche plus les logements, chaque ressort va se détendre et propulser les particules dans le tube. De façon préférentielle, le piston est constitué par un corps cylindrique creux dont la paroi latérale possède au moins une ouverture. Préférentiellement, il existe autant d'ouvertures que de logements de particules et lesdits logements sont répartis uniformément autour du tube en étant alignés et régulièrement espacés. Avantageusement, le tube possède un dispositif d'arrêt et de positionnement du piston situé entre ledit piston et l'extrémité du tube par laquelle les particules sont éjectées. Préférentiellement, le dispositif d'arrêt est conçu pour stopper le piston mu par les gaz, dans une position pour laquelle chaque ouverture de sa paroi latérale vient en correspondance avec chaque logement des particules. Ainsi, sous l'effet de la pression générée par les gaz, le piston se déplace pour se bloquer dans une position permettant aux particules d'envahir une portion du tube. De façon préférentielle, le piston possède un opercule transversal et est positionné dans le tube de façon à ce que l'opercule soit situé en amont des ouvertures, et ledit opercule est calibré pour céder à une pression seuil atteinte par les gaz lorsque le piston est stoppé par le dispostif d'arrêt. Chronologiquement, le fonctionnement d'une seringue selon ce mode de réalisation préféré distingue deux phases : la première au cours de laquelle le piston se déplace et vient se bloquer contre le dispositif d'arrêt, permettant ainsi de libérer les particules dans le tube, et la seconde, intervenant juste après la première, et pendant laquelle la pression des gaz dans l'espace compris entre le générateur de gaz et l'opercule croît, jusqu'à atteindre une valeur seuil entraînant la perforation dudit opercule. La libération soudaine des gaz sous pression par rupture de l'opercule crée une onde de pression assimilable à celle d'un choc. L'onde ainsi émise dans le tube d'éjection met en mouvement les particules sous forme de nuage qui sont éjectées à haute vitesse. Avantageusement, le dispositif d'arrêt est constitué par une couronne fixée à l'intérieur du tube. En effet, la pièce constituant le dispositif d'arrêt doit servir de butée au piston mais ne doit pas empêcher les particules de passer. Cette pièce doit donc être ajourée et peut revêtir aussi, par exemple, la forme d'une grille transversale.

Selon un second mode de réalisation préféré de l'invention, le tube est traversé par un canal transversal dans lequel est logé le piston présentant une partie pleine et une partie creuse contenant les particules et ledit piston, qui est logé dans le canal transversal de façon à obturer initialement le tube avec sa partie pleine, peut se déplacer le long du canal transversal, grâce à un dispositif de poussée, jusqu'à positionner sa partie creuse dans le prolongement du tube. Préférentiellement, le dispositif de poussée est réalisé par prélèvement de gaz grâce à une tubulure reliant la zone de la chambre située à proximité du générateur de gaz au canal transversal. A l'image de ce qui est décrit dans le premier mode de réalisation préféré de l'invention, le dispositif de poussée du piston, et donc des particules, s'appuie sur une source énergétique préexistante permettant de gagner de la place tout en étant particulièrement performante. De façon avantageuse, le canal transversal dispose d'un moyen de blocage pour maintenir le piston dans une position pour laquelle sa partie creuse se retrouve en continuité du tube. Donc, dans un premier temps, sous l'effet des gaz, le piston se déplace pour libérer les particules de principe actif dans le tube et les déconfiner. De façon préférentielle, la partie creuse du piston contenant les particules est initialement fermée par la paroi du canal transversal. Avantageusement, la partie creuse du piston possède un opercule de sorte que, en se déplaçant, le piston ouvre sa partie creuse, de manière à apporter les particules dans le tube et l'opercule est destiné à céder au-delà d'une pression seuil pour expulser les particules. Avantageusement, l'opercule est placé en amont des particules par rapport au sens de propagation des gaz dans la seringue, de façon à se rompre et à créer une onde de choc juste avant d'interagir avec les particules déjà en mouvement. De cette manière, lorsque le piston s'est déplacé et qu'il est bloqué par le moyen de blocage du canal transversal, la seringue présente, successivement et en continuité, le générateur de gaz, une chambre d'expansion des gaz de combustion délimitée par l'opercule du piston, l'opercule, les particules de principe actif qui sont déconfinées et, enfin, le tube d'éjection desdites particules. Une fois la pression seuil atteinte par les gaz, l'opercule cède, et le choc, ainsi créé, accélère brutalement les particules déconfinées situées en aval dudit opercule par rapport au mouvement des gaz, et les éjecte vers la peau du patient. Selon un autre mode de réalisation de l'invention, le piston peut également se présenter sous la forme d'un sous-ensemble initialement étanche, pour lequel, notamment, la partie creuse est fermée par l'intermédiaire de deux languettes amovibles. Pour cette configuration, le déplacement du piston dans le canal transversal, permet de retirer l'une des deux languettes pour libérer préalablement les particules dans le tube, l'autre languette jouant le rôle d'un opercule. La languette peut aussi être déchirée, le but essentiel étant de créer une ouverture pour permettre le déconfinement et la libération avant l'accélération par l'onde de choc des particules dans le tube. Selon un autre mode de réalisation de l'invention, le dispositif de poussée est constitué par un ressort. Avantageusement, le ressort est précontraint entre le piston transporteur de particules bloqué contre une butée amovible, et l'une des extrémités du canal transversal. De façon préférentielle, l'actionnement du déclencheur entraîne, d'une part, l'enlèvement de la butée amovible permettant ainsi le déplacement du piston sous l'effet du ressort qui se détend et, d'autre part, l'activation du générateur de gaz pour provoquer l'arrivée des gaz dans le tube d'éjection. Ces deux actions sont découplées l'une par rapport à l'autre, et doivent se produire avec un écart de temps très faible pour assurer une injection homogène des particules déconfinées.

Les seringues sans aiguille selon l'invention bénéficient des avantages liés à un fonctionnement par choc, en particulier en terme de vitesse d'éjection des particules, tout en assurant un maintien fiable des particules en mode stockage.

De plus, la grande variabilité des compositions pyrotechniques pouvant constituer la source énergétique des seringues, autorise une grande souplesse d'utilisation, en permettant d'ajuster les paramètres du « moteur » de la seringue au cas à traiter.

Enfin, lorsque la poussée des particules dans le tube d'éjection est assurée par prélèvement des gaz de combustion et lorsque le générateur de gaz comprend une charge pyrotechnique de très petite taille, la seringue sans aiguille est dotée d'un mécanisme de fonctionnement très performant mais d'encombrement réduit, lui conférant un caractère fonctionnel particulièrement marqué.

On donne ci-après la description détaillée de plusieurs modes de réalisation préférés de l'invention en se référant aux figures 1 à 7.

La figure 1 est un schéma de principe en coupe axiale longitudinale d'une seringue sans aiguille selon l'invention et dont les particules sont logées dans au moins deux logements inamovibles situés à l'extérieur du conduit du tube d'éjection.

La figure 2 est un schéma en coupe axiale longitudinale du système de retenue des particules de la seringue de la figure 1.

La figure 3 est une vue en coupe axiale longitudinale d'un mode de réalisation préféré de la seringue sans aiguille selon l'invention n'ayant pas encore fonctionné et dont les particules sont logées dans six logements inamovibles situés à l'extérieur du tube d'éjection.

La figure 4 est une vue en coupe axiale longitudinale agrandie de la seringue de la figure 3 après fonctionnement.

La figure 5 est une vue en coupe axiale longitudinale partielle d'une seringue sans aiguille selon l'invention et dont les particules sont logées dans un piston situé dans un canal transversal, la seringue n'ayant pas fonctionné.

La figure 6 représente la seringue de la figure 5 mais ayant fonctionné.

La figure 7 est un schéma en coupe axiale longitudinale du système de retenue des particules de la seringue des figures 5 et 6.

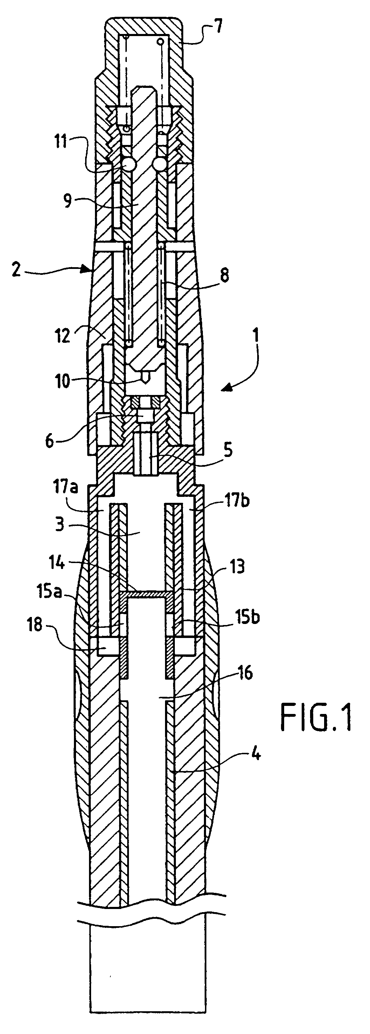

En se référant à la figure 1, une seringue sans aiguille 1, selon le premier mode de réalisation préféré de l'invention, comprend successivement un générateur de gaz pyrotechnique 2, une chambre d'expansion 3, un système de maintien des particules et un tube d'éjection 4 desdites particules destiné à venir en appui contre la peau du patient à traiter.

Le générateur pyrotechnique 2 de gaz comprend un dispositif d'initiation d'une charge pyrotechnique 5 faisant intervenir un dispositif de percussion et une amorce 6. Le dispositif de percussion qui est déclenché par un bouton poussoir 7 comprend un ressort 8 et une masselotte 9 allongée munie d'un percuteur 10. La masselotte 9 est bloquée par au moins une bille 11 de maintien coincée entre ladite masselotte 9 et un corps cylindrique creux 12 dans lequel est susceptible de se déplacer ladite masselotte 9. L'amorce 6 et la charge pyrotechnique 5, de forme sensiblement cylindrique, sont logées dans le corps cylindrique creux 12, en aval de la masselotte 9. La charge pyrotechnique 5, qui est logée dans le corps creux 12, présente une face circulaire plane débouchant sur un espace libre de la seringue qui constitue la chambre d'expansion 3 des gaz qui seront issus de la combustion de la charge pyrotechnique 5.

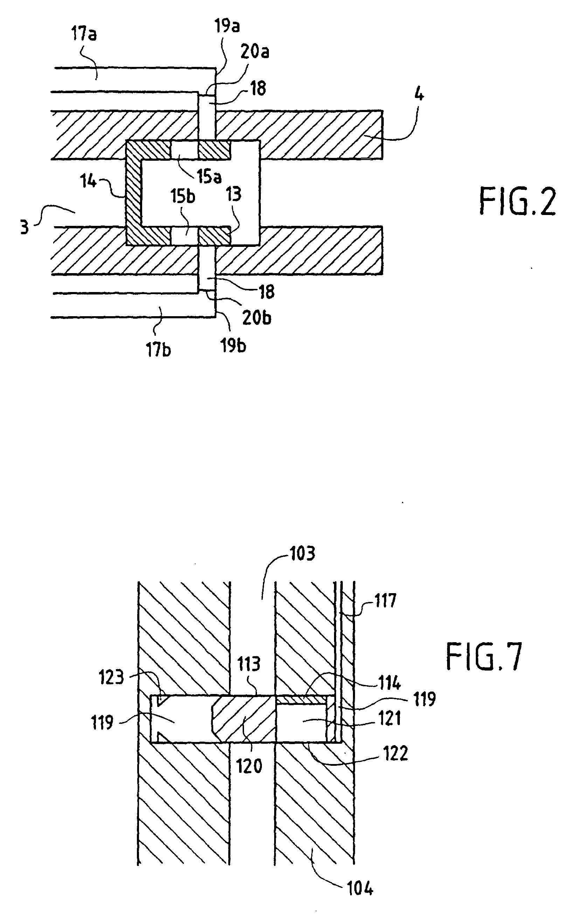

En se référant à la figure 2, cette chambre 3, de forme sensiblement cylindrique est délimitée à son extrémité opposée à celle constituée par la charge pyrotechnique 5, par un piston 13 composé d'un corps cylindrique creux, de section droite, dont l'une des extrémités est libre et l'autre est fermée par un opercule 14 susceptible de se rompre au delà d'une pression seuil dans ladite chambre 3.

De façon plus précise, c'est l'opercule 14 plan du piston 13 qui délimite la longueur de la chambre 3. Le corps cylindrique creux dudit piston 13 possède deux ouvertures 15a, 15b diamétralement opposées. Le piston 13 est positionné dans la seringue 1 de sorte que son extrémité libre est située du côté du tube d'éjection 4 et l'opercule 14 est placé en amont de ladite extrémité libre par rapport au sens de propagation des gaz issus de la combustion de la charge pyrotechnique 5. La paroi latérale externe du piston 13 est au contact de la paroi latérale interne d'un canal 16 qui prolonge la chambre 3, ledit canal 16 étant lui-même prolongé par le conduit du tube d'éjection 4. La chambre 3, le canal intermédiaire 16 et le tube d'éjection 4 sont de forme cylindrique, ladite chambre 3 et ledit conduit du tube 4 ayant le même diamètre et le canal intermédiaire 16 ayant un diamètre supérieur. Ces trois éléments, en continuité les uns des autres, sont délimités par des épaulements internes marquant leur différence de diamètre. Deux tubulures 17a, 17b situées dans l'épaisseur de la seringue 1 et parallèles à l'axe de la chambre 3, relient, chacune, la chambre 3 au canal intermédiaire 16. Plus précisément, chacune des tubulures 17a, 17b prend naissance dans la chambre 3, dans une zone très proche de la charge pyrotechnique 5, et se termine approximativement dans la partie médiane du canal intermédiaire 16. Le piston 13, qui est situé dans le canal 16, est bloqué contre l'épaulement interne marquant la frontière entre la chambre 3 et ledit canal intermédiaire 16, et son corps cylindrique creux obture l'extrémité des deux tubulures 17a, 17b débouchant dans le canal 16 de manière à ce que les deux ouvertures 15a, 15b soient situées entre l'opercule 14 et la partie du corps cylindrique creux du piston 13 obturant les deux tubulures 17a, 17b. Ces deux tubulures 17a, 17b, qui sont parallèles à l'axe de la chambre 3, présentent, chacune, un coude leur permettant de comporter un petit segment terminal 19a, 19b débouchant dans le canal 16, perpendiculairement à son axe. Le principe actif, pulvérulent ou sous forme de poudre sèche, occupe chacun des deux petits segments 19a, 19b des tubulures 17a, 17b, dans un espace 18 délimité, d'une part, par la paroi latérale du piston 13 et, d'autre part, par une membrane poreuse 20a, 20b disposée transversalement dans chaque tubulure 17a, 17b.
Le tube d'éjection 4 des particules de principe actif a le même diamètre que celui de la chambre 3 et peut, avantageusement, se terminer par un bourrelet amortissant pour faciliter le contact de la seringue 1 sur la peau du patient.

Le mode de fonctionnement de ce premier mode de réalisation préféré de l'invention s'effectue comme suit.

L'utilisateur positionne la seringue 1 de façon à ce que l'extrémité du tube d'éjection 4 vient en appui contre la peau du patient à traiter.

Une pression sur le bouton poussoir 7 permet, d'une part, au corps cylindrique creux 12 de se déplacer jusqu'à ce que sa partie évasée se présente en face de la bille 11 de maintien et, d'autre part, de comprimer le ressort 8. La bille 11 sort de son logement, libérant alors la masselotte 9, qui, soumise à l'action du ressort 8 qui se détend, est brutalement accélérée vers l'amorce 6, le percuteur 10 en avant. La réaction de l'amorce 6 entraîne la mise à feu de la charge pyrotechnique 5 qui émet des gaz envahissant simultanément la chambre d'expansion 3 et les deux tubulures 17a, 17b. Lorsque la pression dans la chambre 3 atteint un niveau seuil, le piston 13 se déplace linéairement dans le canal intermédiaire 16 jusqu'à venir en butée contre l'épaulement interne marquant la frontière entre ledit canal 16 et le tube d'éjection 4. Cette position finale du piston 13 correspond à la mise en continuité de ses deux ouvertures 15a, 15b avec le petit segment terminal 19a, 19b de chaque tubulure 17a, 17b.

Avant que le piston 13 n'arrive en butée, le principe actif est confiné et commence à se répandre dans le tube d'éjection 4 grâce notamment à la poussée des gaz se trouvant dans les tubulures 17a, 17b. Une fois le piston 13 arrivé en butée, le principe actif se retrouve intégralement en mouvement dans le tube d'éjection 4 tandis que la pression dans la chambre 3 continue de croître très rapidement. Lorsque la pression atteint une valeur seuil, l'opercule 14 finit par céder, libérant ainsi une onde de choc qui rejoint, emporte et accélère les particules encore sous la forme d'un nuage diffus. La libération des particules dans le tube 4 et la rupture de l'opercule 14 doivent s'effectuer sur un intervalle de temps très court, de l'ordre d'une milliseconde ou de quelques millisecondes, pour que les particules n'aient pas eu le temps de se regrouper entre elles.

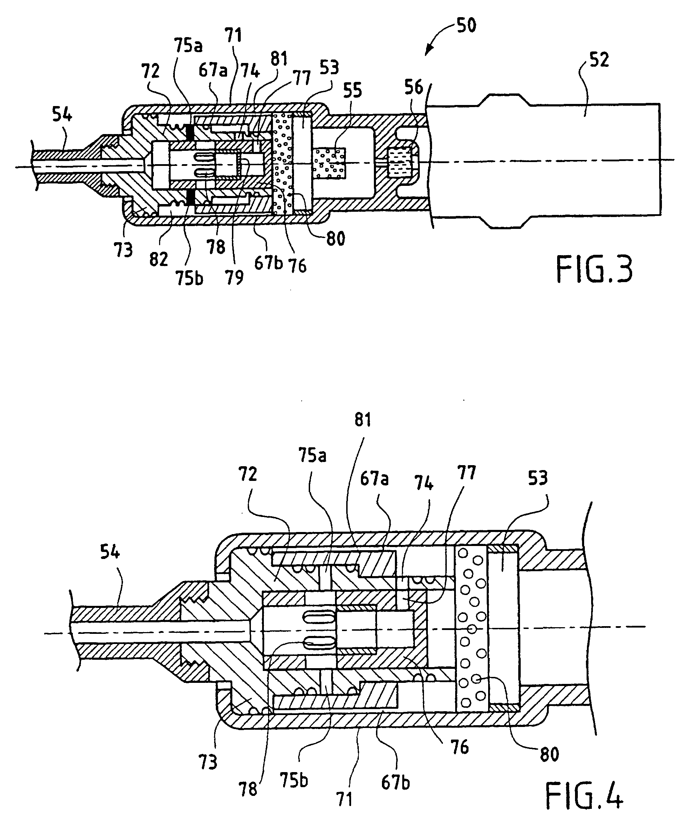

En se référant aux figures 3 et 4, une seringue sans aiguille 50 selon une autre version du premier mode de réalisation de l'invention comprend également un générateur pyrotechnique 52 de gaz, une chambre d'expansion 53, un système de maintien des particules et un tube d'éjection 54 desdites particules destiné à venir en appui contre la peau du patient à traiter.

Le générateur pyrotechnique 52 de gaz comprend un dispositif d'initiation d'une charge pyrotechnique 55 faisant intervenir un dispositif de percussion et une amorce 56. Les caractéristiques du dispositif de percussion, non représenté sur les figures 3 et 4, sont identiques à celles du dispositif de percussion décrit pour le premier mode de réalisation de l'invention. Le générateur pyrotechnique 52, dont une extrémité est délimitée par la charge pyrotechnique 55, est prolongé, du côté de ladite extrémité, par un corps cylindrique creux 71, lui-même prolongé par le tube d'éjection 54. L'extrémité dudit corps 71, située du côté du tube 54, a été rabattue vers l'intérieur en faisant un angle droit, de sorte qu'elle présente une ouverture centrale de diamètre inférieur à celui du corps 71. En continuité du générateur de gaz 52, le corps creux 71 présente successivement, la chambre 53 de forme sensiblement cylindrique, un filtre 80 transversal fixé audit corps 71 et le système de maintien des particules de principe actif comprenant une pièce inamovible creuse 72 sensiblement cylindrique, bloquée entre le filtre 80 et l'extrémité rabattue du corps creux 71. La pièce creuse 72 possède un canal central cylindrique de diamètre constant prolongé par une partie convergente débouchant dans le tube d'éjection 54. L'extrémité libre dudit canal central cylindrique de diamètre constant est obturée par le filtre 80. Le diamètre externe de ladite pièce 72 est inférieur au diamètre interne du corps creux 71 de sorte qu'il subsiste un espace entre ces deux éléments. Ladite pièce creuse 72 possède une embase cylindrique élargie 73 dont la paroi externe est au contact de la paroi interne du corps creux 71, ladite embase 73 se retrouvant en butée contre l'extrémité rabattue dudit corps 71. Cette pièce 72 inamovible présente, dans sa partie amont, une série de premiers orifices 74 (dont un seul est représenté) traversant sa paroi latérale, et, dans sa partie située plus en aval, six autres orifices 75a, 75b diamétralement opposés et traversant également sa paroi latérale, ces orifices 75a, 75b diamétralement opposés étant destinés à loger les particules solides de principe actif. Le canal central de la pièce 72 inamovible loge un piston 76 représenté par un corps cylindrique creux, dont la paroi latérale externe est au contact de la paroi latérale interne dudit canal, et présentant une extrémité fermée au contact du filtre 80 et une autre extrémité ouverte. Dans une zone amont, la paroi latérale du piston 76 est traversée par une série d'orifices 77 (dont un seul est représenté) et dans une zone aval, ledit piston 76 présente six ouvertures 78 traversant également sa paroi latérale, lesdites ouvertures 78 étant alignées les unes par rapport aux autres et uniformément réparties sur la périphérie dudit piston 76. La longueur du piston 76 est inférieure à la longueur du canal central de la pièce 72, et le piston 76 est placé dans ledit canal de sorte que son extrémité fermée soit au contact de la surface du filtre 80. Le canal interne du piston 76 présente, en continuité l'une de l'autre, une partie amont et une partie aval séparées par un opercule 79 transversal présentant des lignes de fragilisation permettant de définir un motif étoilé, ledit opercule 79 étant solidaire dudit piston 76. Les orifices 77 débouchent dans la partie amont du canal du piston 76 et les ouvertures 78 débouchent dans la partie aval dudit canal. L'espace compris entre le corps cylindrique creux 71 de la seringue 50 et la pièce cylindrique creuse 72 inamovible est partiellement occupée par une pièce cylindrique 81 en forme de couronne, dont la longueur est inférieure à la longueur dudit espace délimitée par l'embase élargie 73 de ladite pièce creuse 72 et le filtre 80 , et possédant à sa périphérie au moins deux rainures rectilignes longitudinales, parallèles entre elles et à son axe de révolution, de façon à constituer entre la paroi latérale externe de ladite pièce cylindrique 81 et la paroi latérale interne du corps cylindrique creux 71 deux tubulures 67a, 67b. Ladite pièce 81 en forme de couronne est positionnée contre le filtre 80, permettant l'existence d'un espace libre 82 délimité, d'une part, par la paroi latérale externe de la pièce creuse 72 inamovible et la paroi latérale interne du corps cylindrique creux 71 et, d'autre part, par l'embase élargie 73 de ladite pièce creuse 72 et l'une des extrémité de la pièce 81 en forme de couronne. Les différentes pièces décrites ci avant sont agencées les unes par rapport aux autres de sorte que les orifices 75a, 75b, destinés à recevoir les particules de principe actif, d'une part, sont obturés par la paroi latérale du piston 76 située en aval des ouvertures 78, et, d'autre part, débouchent dans l'espace libre 82 situé entre la pièce 81 en forme de couronne et l'embase élargie 73 de la pièce 72 creuse inamovible. Les particules de principe actif sont logées dans ces orifices 75a, 75b entre le piston 76 et une membrane poreuse qui affleure la surface latérale externe de la pièce 72 creuse.

Avantageusement, un opercule secondaire transversal est disposé à l'extrémité du tube d'éjection 54 qui jouxte la partie convergente du canal central de la pièce cylindrique creuse 72.

Le principe de fonctionnement de cette seconde version du premier mode de réalisation préféré de la seringue s'effectue comme suit.

L'étape qui permet d'aboutir à la mise à feu de la charge pyrotechnique 55 par l'utilisateur est rigoureusement identique à celle décrite précédemment pour le premier mode de réalisation préféré de l'invention. Les gaz alors émis par le combustion de la charge pyrotechnique 55 envahissent d'abord la chambre 53, puis les tubulures 67a, 67b pour venir occuper l'espace libre 82 situé entre la pièce cylindrique creuse 72 et ledit corps creux 71. Les gaz s'accumulant dans cet espace 82 font alors pression sur la paroi poreuse qui obture les orifices 75a, 75b logeant les particules de principe actif. Dans un deuxième temps, le piston 76 se déplace dans le canal central jusqu'à venir faire correspondre, d'une part, ses six ouvertures 78 avec les six orifices 75a, 75b contenant les particules de manière à libérer les particules dans ledit piston 76 et, d'autre part, ses orifices 77 situés dans sa zone amont avec les orifices 74 de la partie amont de la pièce cylindrique creuse 72 pour permettre aux gaz d'envahir la partie amont du canal interne du piston 76, ladite partie amont étant délimitée par l'opercule 79. Le piston 76 finit sa course en venant en butée contre un épaulement interne du canal central de la pièce cylindrique creuse 72. Chronologiquement, lors du déplacement dudit piston 76, la phase de mise en correspondance des ouvertures 78 avec les orifices 75a, 75b contenant les particules intervient juste avant la phase de mise en correspondance des autres orifices 74, 77, de manière à ce que les particules aient déjà commencé a être libérées juste avant la montée en pression de la partie amont du canal interne du piston 76. Une fois que la pression atteint une valeur seuil dans ladite partie amont du piston 76, l'opercule 79 s'ouvre en pétales sans se fragmenter, et génère une onde de choc qui entraîne les particules de principe actif, d'abord dans le tube d'éjection 54 puis vers la peau du patient à traiter. L'opercule secondaire, avantageusement positionné à la naissance du tube d'éjection 54, sert à maintenir temporairement, dans la seringue 50, les particules qui ont été soufflées de leurs orifices 75a, 75b, ledit opercule ne présentant aucun caractère de résistance vis à vis de l'onde de choc émise en amont et s'ouvrant également en pétales.

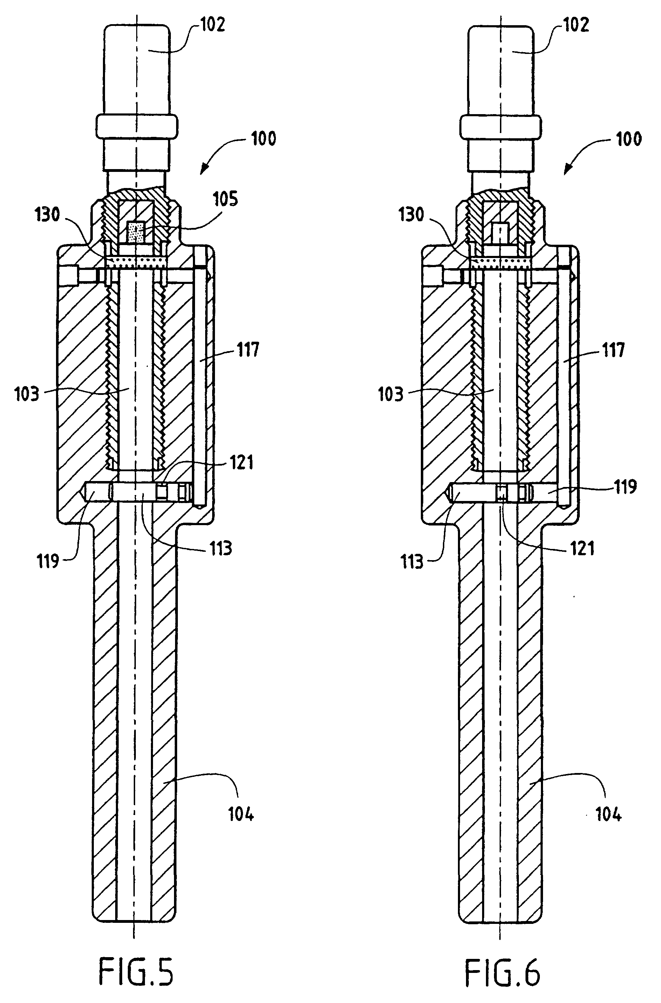

En se référant aux figures 5, 6 et 7, une seringue sans aiguille 100 selon le deuxième mode de réalisation préféré de l'invention, comprend successivement un générateur de gaz pyrotechnique 102, une chambre d'expansion 103, un système de maintien des particules et un tube d'éjection 104 desdites particules destiné à venir en appui contre la peau du patient à traiter. Le générateur de gaz pyrotechnique 102, dont seul le contour et la charge pyrotechnique 105 ont été représentés sur les figures 5 et 6 et qui fonctionne à partir d'un dispositif de percussion, une charge pyrotechnique 105 et une amorce, est en tout point identique à celui décrit ci-avant pour le premier mode de réalisation préféré de l'invention. La charge pyrotechnique 105 présente une face annulaire plane débouchant sur la chambre d'expansion 103. La chambre d'expansion 103 et le tube d'éjection 104 ont le même diamètre et leur frontière est marquée par un canal transversal 119 sensiblement cylindrique, dont une extrémité est plane et fermée, et l'autre extrémité, également plane, se prolonge par une tubulure 117 débouchant dans ladite chambre 103, dans une zone très proche de la charge pyrotechnique 105 et juste en aval d'un filtre transversal 130 destiné à filtrer les particules solides et à refroidir les gaz. Le canal transversal 119 est en partie occupé par un piston 113 de forme sensiblement cylindrique, présentant, en continuité l'une de l'autre, une partie pleine 120 et une partie creuse 121, de même diamètre, ledit piston 113 étant délimité, suivant l'axe du canal transversal 119, par deux faces circulaires planes. La partie creuse 121 du piston 113, qui est destinée à contenir le principe actif pulvérulent, a une forme sensiblement cylindrique et est délimitée, suivant l'axe du canal 119, par une face circulaire plane appartenant à la partie pleine 120 et une face circulaire plane représentant l'une des deux extrémités du piston 113. De plus, cette partie creuse 121 présente, sur sa paroi latérale et en des positions diamétralement opposées par rapport à l'axe de ladite partie creuse 121, un opercule 114 apte à se rompre au delà d'une pression seuil et une ouverture 122 qui rend ladite partie creuse 121 assimilable à un espace ouvert.

L'opercule 114 ainsi que l'ouverture 122 ont une longueur, suivant l'axe du canal transversal 119, inférieure au diamètre de la chambre 103 et sont alignés de façon à présenter le même axe de symétrie. La longueur de la partie creuse 121, prise suivant son axe de symétrie, reste inférieure à l'épaisseur de la paroi latérale de la seringue 100, et la longueur totale du piston 113, suivant son axe, est légèrement supérieure à la longueur représentée par la somme de la longueur de la partie du canal 119 se terminant par la tubulure 117 et du diamètre de la chambre 103. Le piston 113 est enfoncé dans la partie du canal transversal 119 se terminant par la tubulure 117, de manière à ce que, d'une part, sa partie pleine 120 isole parfaitement la chambre 103 du tube d'éjection 104, en débordant de chaque côté de ladite chambre 103 ou dudit tube 104 et, d'autre part, sa partie creuse 121 se retrouve incluse intégralement dans la partie du canal 119 se terminant par la tubulure 117. Le piston 113 est monté coulissant à force dans le canal 119, de sorte que la paroi latérale externe dudit piston 113 est au contact de la paroi interne dudit canal 119. De cette manière, la partie creuse 121 du piston 113 est fermée puisque la paroi interne du canal 119 ferme l'ouverture 122. Le piston 113 occupe le canal 119 dans une position telle que si il était translaté dans ledit canal 119 jusqu'à présenter sa partie creuse 121 en continuité de la chambre 103 et du tube d'éjection 104, sans subir d'autres mouvements, l'opercule 114 et l'ouverture 122 se retrouveraient perpendiculaires à l'axe de la chambre 103 et du tube 104, et l'opercule 114 serait en amont de l'ouverture 122 par rapport au sens de propagation des gaz en provenance de la charge pyrotechnique 105. Un espace libre subsiste entre le piston 113 et le fond de la partie du canal 119 dans laquelle il est enfoncé, ledit espace étant en communication avec la tubulure 117. La partie inoccupée du canal 119 se terminant par une extrémité plane et fermée, présente un étranglement 123 à proximité de ladite extrémité. Enfin, le tube d'éjection 104 peut également se terminer par un bourrelet amortissant pour faciliter le contact de la seringue 100 sur la peau du patient.

Le mode de fonctionnement de ce deuxième mode de réalisation préféré de l'invention se déroule comme suit. L'étape qui permet d'aboutir à la mise à feu de la charge pyrotechnique 105 par l'utilisateur est identique à celle décrite précédemment pour le premier mode de réalisation préféré de l'invention. Les gaz alors émis par la combustion de la charge pyrotechnique 105 envahissent simultanément la chambre 103, délimitée par la partie pleine 120 du piston 113, et la tubulure 117. Les gaz s'engouffrent dans l'espace libre compris entre ledit piston 113 et l'extrémité plane de la partie du canal 119 dans laquelle il est enfoncé. En se référant à la figure 6, cet espace s'expanse sous l'effet de la pression, en déplaçant le piston 113 qui vient se bloquer dans l'étranglement 123 du canal 119. Dans cette position terminale, le piston 113 aligne alors sa partie creuse 121 sur la chambre 103 et le tube 104. En amorçant son déplacement, le piston 113 avait déjà commencé à ouvrir sa partie creuse 121 puisque la paroi interne du canal 119 n'obturait plus intégralement ladite partie creuse 121, libérant ainsi les particules solides de principe actif dans le tube d'éjection 104. Une fois bloqué par l'étranglement 123, le piston 113 continue d'obturer momentanément la chambre 103 dont le niveau de pression croit. Une fois que ce niveau de pression atteint une valeur seuil, l'opercule 114 cède, créant une onde de choc qui emporte et accélère les particules encore sous la forme d'un nuage diffus. La libération des particules dans le tube 104 et la rupture de l'opercule 114 doivent s'effectuer sur un intervalle de temps très court, de l'ordre de quelques millisecondes, pour que les particules n'aient pas eu le temps de se regrouper entre elles.

## Revendications

1. Seringue sans aiguille (1,50, 100) comprenant un déclencheur, un générateur de gaz prolongé par une chambre d'expansion (3, 53, 103) des gaz, un piston (13, 76, 113), un tube d'éjection (4, 54, 104), lesdites particules étant logées à l'extérieur dudit tube (4, 54,104) et les gaz, émis par ledit générateur, provoquant l'arrivée des particules dans le tube (4, 54, 104) par déplacement dudit piston (13, 76, 113), puis leur mise en vitesse dans ledit tube (4, 54, 104) **caractérisée en ce que** le piston (13, 76, 113) possède un opercule (14, 79, 114) calibré, de sorte que ledit opercule (14, 79, 114) se retrouve en amont des particules libérées dans le tube (4, 54, 104) et contribue à créer, en cédant au-delà d'une pression seuil, une onde de choc destinée à emporter lesdites particules.

2. Seringue sans aiguille selon la revendication 1, **caractérisée en ce que** le générateur de gaz est un générateur pyrotechnique (2, 52, 102) comprenant une charge pyrotechnique (5, 55, 105) et un dispositif d'initiation.

3. seringue sans aiguille selon la revendication 1, **caractérisée en ce qui** les particules sont situées dans au moins un logement inamovible (18, 75a, 75b) extérieur au conduit du tube (4, 54), chaque logement (18, 75a, 75b) étant obturé par le piston (13, 76) positionné dans ledit tube (4, 54), ledit piston (13, 76) pouvant se déplacer, sous l'effet des gaz, pour ouvrir chaque logement (18, 75a, 75b) et libérer les particules dans le tube (4, 54).

4. Seringue sans aiguille selon la revendication 3, **caractérisée en ce qu'**un dispositif de propulsion permet aux particules libérées par le piston (13, 76) d'être poussées dans le tube (4, 54) à partir de leur logement (18, 75a, 75b).

5. Seringue sans aiguille selon la revendication 4, **caractérisée en ce que** le dispositif de propulsion est réalisé par prélèvement de gaz grâce à au moins une tubulure (17a, 17b, 67a, 67b) reliant la zone de la chambre (3, 53) située à proximité du générateur de gaz aux logements (18, 75a, 75b) des particules.

6. Seringue sans aiguille selon la revendication 5, **caractérisée en ce que** les particules sont logées dans la partie terminale de chaque tubulure (17a, 17b, 67a, 67b), contre le piston (13,76).

7. Seringue sans aiguille selon la revendication 3, **caractérisée en ce que** le piston (13, 76) est constitué par un corps cylindrique creux dont la paroi latérale possède au moins une ouverture (15a, 15b, 78).

8. Seringue sans aiguille selon la revendication 7, **caractérisée en ce que** le tube (4, 54) possède un dispositif d'arrêt et de positionnement du piston (13, 76) situé entre ledit piston (13, 76) et l'extrémité du tube (4, 54) par laquelle les particules sont éjectées, chaque ouverture (15a, 15b, 78) de la paroi latérale du piston (13, 76) venant en correspondance avec chaque logement (18, 75a, 75b) des particules.

9. Seringue sans aiguille selon la revendication 8, **caractérisée en ce que** le piston (13, 76) est positionné dans le tube de façon à ce que l'opercule (14, 79) soit situé en amont des ouvertures (15a, 15b, 78) et ledit opercule (14, 79) est calibré pour céder à une pression seuil atteinte par les, gaz lorsque le piston (13, 76) est stoppé par le dispositif d'arrêt.

10. seringue sans aiguille selon la revendication 1, **caractérisée en ce que** le tube (104) est traversé par un canal transversal (119) dans lequel est logé le piston (113) présentant une partie pleine (120) et une partie creuse (121) contenant les particules, et ledit piston (113), qui est logé dans le canal transversal (119) de façon à obturer initialement le tube (104) avec sa partie pleine (120), peut se déplacer le long du canal transversal (119), grâce à un dispositif de poussée, jusqu'à positionner sa partie creuse (121) dans le prolongement du tube (104).

11. Seringue sans aiguille selon la revendication 10, **caractérisée en ce que** le dispositif de poussée est réalisé par prélèvement de gaz grâce à une tubulure (117) reliant la zone de la chambre (103) située à proximité du générateur de gaz au canal transversal (119).

12. Seringue sans aiguille selon la revendication 10, **caractérisée en ce que** le canal transversal (119) dispose d'un moyen de blocage (123) pour maintenir le piston (113) dans une position pour laquelle sa partie creuse (121) se retrouve en continuité du tube (104).

13. Seringue sans aiguille selon la revendication 10, **caractérisée en ce que** la partie creuse (121) du piston (113) contenant les particules est initialement fermée par la paroi du canal transversal (119).

14. Seringue sans aiguille selon l'une quelconque des revendications 10 ou 13, **caractérisée en ce que** l'opercule est situé au niveau de la partie creuse (121) du piston (113) de sorte que, en se déplaçant, le piston (113) ouvre sa partie creuse (121), de manière à apporter les particules dans le tube (104) et l'opercule (114) est destiné à céder au-delà d'une pression seuil pour expulser les particules.

15. Seringue sans aiguille selon la revendication 10, **caractérisée en ce qui** le dispositif de poussée est constitué par un ressort.

## Patentansprüche

1. Nadellose Spritze (1, 50, 100), die einen Auslöser, einen durch eine Expansionskammer (3, 53, 103) für die Gase verlängerten Gasgenerator, einen Kolben (13, 76, 113), ein Ausstoßrohr (4, 54, 104) aufweist, wobei die Teilchen sich außerhalb des Rohrs (4, 54, 104) befinden und die vom Generator ausgegebenen Gase die Ankunft der Teilchen im Rohr (4, 54, 104) durch Verschieben des Kolbens (13, 76, 113) und dann ihre Beschleunigung im Rohr (4, 54 104) bewirken, **dadurch gekennzeichnet, dass** der Kolben (13, 76, 113) eine kalibrierte Abdeckfolie (14, 79, 104) aufweist, derart, dass die Abdeckfolie (14, 79, 104) sich stromaufwärts vor den im Rohr (4, 54, 104) freigesetzten Teilchen befindet und dazu beiträgt, indem sie jenseits eines Schwellendrucks nachgibt, eine Stoßwelle zu erzeugen, die dazu bestimmt ist, die Teilchen mitzureißen.

2. Nadellose Spritze nach Anspruch 1, **dadurch gekennzeichnet, dass** der Gasgenerator ein pyrotechnischer Generator (2, 52, 102) ist, der eine pyrotechnische Ladung (5, 55, 105) und eine Zündvorrichtung aufweist.

3. Nadellose Spritze nach Anspruch 1, **dadurch gekennzeichnet, dass** die Teilchen sich in mindestens einem nicht entfernbaren Sitz (18, 75a, 75b) außerhalb des Kanals des Rohrs (4, 54) befinden, wobei jeder Sitz (18, 75a, 75b) vom Kolben (13, 76) verschlossen wird, der im Rohr (4, 54) positioniert ist, wobei der Kolben (13, 76) sich unter der Wirkung der Gase verschieben kann, um jeden Sitz (18, 75a, 75b) zu öffnen und die Teilchen in das Rohr (4, 54) freizusetzen.

4. Nadellose Spritze nach Anspruch 3, **dadurch gekennzeichnet, dass** eine Antriebseinrichtung es ermöglicht, die vom Kolben (13, 76) freigesetzten Teilchen ausgehend von ihrem Sitz (18, 75a, 75b) in das Rohr zu stoßen.

5. Nadellose Spritze nach Anspruch 4, **dadurch gekennzeichnet, dass** die Antriebseinrichtung durch Entnahme von Gas mit Hilfe mindestens eines Rohransatzes (17a, 17b, 67a, 67b) erhalten wird, der die Zone der Kammer (3, 53), die sich in der Nähe des Gasgenerators befindet, mit den Sitzen (18, 75a, 75b) der Teilchen verbindet.

6. Nadellose Spritze nach Anspruch 5, **dadurch gekennzeichnet, dass** die Teilchen im Endbereich jedes Rohransatzes (17a, 17b, 67a, 67b) gegen den Kolben (13, 76) angeordnet sind.

7. Nadellose Spritze nach Anspruch 3, **dadurch gekennzeichnet, dass** der Kolben (13, 76) aus einem zylindrischen Hohlkörper besteht, dessen Seitenwand mindestens eine Öffnung (15a, 15b, 78) aufweist.

8. Nadellose Spritze nach Anspruch 7, **dadurch gekennzeichnet, dass** das Rohr (4, 54) eine Vorrichtung zum Anhalten und Positionieren des Kolbens (13, 76) aufweist, die sich zwischen dem Kolben (13, 76) und dem Ende des Rohrs (4, 54) befindet, durch das die Teilchen ausgestoßen werden, wobei jede Öffnung (15a, 15b, 78) der Seitenwand des Kolbens (13, 76) mit jedem Sitz (18, 75a, 75b) der Teilchen in Übereinstimmung kommt.

9. Nadellose Spritze nach Anspruch 8, **dadurch gekennzeichnet, dass** der Kolben (13, 76) derart im Rohr positioniert ist, dass die Abdeckfolie (14, 79) sich stromaufwärts vor den Öffnungen (15a, 15b, 78) befindet, und dass die Abdeckfolie (14, 79) so kalibriert ist, dass sie einem Schwellendruck nachgibt, der von den Gasen erreicht wird, wenn der Kolben (13, 76) von der Haltevorrichtung angehalten wird.

10. Nadellose Spritze nach Anspruch 1, **dadurch gekennzeichnet, dass** das Rohr (104) von einem Querkanal (119) durchquert wird, in dem der Kolben (113) angeordnet ist, der einen massiven Teil (120) und einen die Teilchen enthaltenden, hohlen Teil (121) aufweist, und dass der Kolben (113), der im Querkanal (119) angeordnet ist, um ursprünglich das Rohr (104) mit seinem massiven Teil (120) zu verschließen, sich mit Hilfe einer Schubeinrichtung so weit entlang des Querkanals (119) verschieben kann, bis sein hohler Teil (121) in der Verlängerung des Rohrs (104) positioniert ist.

11. Nadellose Spritze nach Anspruch 10, **dadurch gekennzeichnet, dass** die Schubeinrichtung durch Entnahme von Gas mit Hilfe eines Rohransatzes (117) erhalten wird, der die Zone der Kammer (103), die sich in der Nähe des Gasgenerators befindet, mit dem Querkanal (119) verbindet.

12. Nadellose Spritze nach Anspruch 10, **dadurch gekennzeichnet, dass** der Querkanal (119) über ein Blockiermittel (123) verfügt, um den Kolben (113) in einer Position zu halten, in der sein hohler Teil (121) sich in kontinuierlicher Verlängerung des Rohrs (104) befindet.

13. Nadellose Spritze nach Anspruch 10, **dadurch gekennzeichnet, dass** der die Teilchen enthaltende, hohle Teil (121) des Kolbens (113) ursprünglich von der Wand des Querkanals (119) verschlossen wird.

14. Nadellose Spritze nach einem der Ansprüche 10 oder 13, **dadurch gekennzeichnet, dass** die Abdeckfolie sich in Höhe des hohlen Teils (121) des Kolbens (113) befindet, so dass der Kolben (113) bei seiner Verschiebung seinen hohlen Teil (121) öffnet, um die Teilchen in das Rohr (104) zu bringen, und dass die Abdeckfolie (114) dazu bestimmt ist, jenseits eines Schwellendrucks nachzugeben, um die Teilchen auszustoßen.

15. Nadellose Spritze nach Anspruch 10, **dadurch gekennzeichnet, dass** die Schubeinrichtung von einer Feder gebildet wird.

## Claims

1. Needleless syringe (1, 50, 100) comprising a trigger, a gas generator extended by an expansion chamber (3, 53, 103) for gases, a piston (13, 76, 113) and an ejection tube (4, 54, 104), the said particles being housed outside the said tube (4, 54, 104) and the gases, emitted by the said generator, bringing about the arrival of the particles in the tube (4, 54, 104) by displacement of the said piston (13, 76, 113) and then their acceleration in the said tube (4, 54, 104) **characterized in that** the piston (13, 76, 113) possesses a calibrated closure (14, 79, 114), so that the said closure (14, 79, 114) is situated upstream to the particles released in the tube (4, 54, 104) and contributes to the creation, by yielding above a threshold pressure, of a shock wave designed to carry the said particles.

2. Needleless syringe according to claim 1, **characterised in that** the gas generator is a pyrotechnic generator (2, 52, 102) comprising a pyrotechnic charge (5, 55, 105) and an initiating device.

3. Needleless syringe according to claim 1, **characterised in that** the particles are situated in at least one non-detachable housing (18, 75a, 75b) outside the conduit of the tube (4, 54), each housing (18, 75a, 75b) being closed by the piston (13, 76) positioned in the said tube (4, 54), the said piston (13, 76) being capable of moving, under the effect of the gases, in order to open each housing (18, 75a, 75b) and to liberate the particles in the tube (4, 54).

4. Needleless syringe according to claim 3, **characterised in that** a propulsion device enables the particles released by the piston (13, 76) to be pushed into the tube (4, 54) from their housing (18, 75a, 75b).

5. Needleless syringe according to claim 4, **characterised in that** the propulsion device is produced by taking off gas by means of at least one pipe (17a, 17b, 67a, 67b) connecting the zone of the chamber (3, 53), situated close to the gas generator, to the housings (18, 75a, 75b) for the particles.

6. Needleless syringe according to claim 5, **characterised in that** the particles are housed in the end part of each pipe (17a, 17b, 67a, 67b) against the piston (13, 76) .

7. Needleless syringe according to claim 3, **characterised in that** the piston (13, 76) consists of a hollow cylindrical body of which the lateral wall possesses at least one opening (15a, 15b, 78).

8. Needleless syringe according to claim 7, **characterised in that** the tube (4, 54) possesses a device for stopping and positioning the piston (13, 76) situated between the said piston (13, 76) and the end of the tube (4, 54) through which the particles are ejected, each opening (15a, 15b, 78) of the lateral wall of the piston (13, 76) coming into correspondence with each housing (18, 75a, 75b) for the particles.

9. Needleless syringe according to claim 8, **characterised in that** the piston (13, 76) is positioned in the tube so that the closure (14, 79) is situated upstream to the openings (15a, 15b, 78) and the said closure (14, 79) is calibrated so as to yield at a threshold pressure reached by the gases when the piston (13, 76) is stopped by the stopping device.

10. Needleless syringe according to claim 1, **characterised in that** the tube (104) is traversed by a transverse channel (119) in which the piston (113) is housed having a solid part (120) and a hollow part (121) containing the particles, and the said piston (113), which is housed in the transverse channel (119) so as initially to close off the tube (104) with its solid part (120) can move along the transverse channel (119) by virtue of a thrust device, until its hollow part (121) is positioned in the extension of the tube (104) .

11. Needleless syringe according to claim 10, **characterised in that** the thrust device is produced by drawing off gas by means of a pipe (117) connecting the zone of the chamber (103), situated close to the gas generator, to the transverse channel (119).

12. Needleless syringe according to claim 10, **characterised in that** the transverse channel (119) possesses a locking means (123) for holding the piston (113) in a position for which its hollow part (121) is situated in the continuity of the tube (104).

13. Needleless syringe according to claim 10, **characterised in that** the hollow part (121) of the piston (113) containing the particles is initially closed by the wall of the transverse channel (119).

14. Needleless syringe according to either of claims 10 or 13, **characterized in that** the closure is situated at the level of the hollow part (121) of the piston (113) so that, as it moves, the piston (113) opens its hollow part (121) so as to bring the particles into the tube (104) and the closure (114) is designed to yield above a threshold pressure in order to expel the particles.

15. Needleless syringe according to claim 10, **characterised in that** the thrust device consists of a spring.
